# FASCICULE DE BREVET EUROPEEN

(11) **EP 2 218 952 B1**
(45) Date de publication et mention de la délivrance du brevet: **27.02.2013**
(21) Numéro de dépôt: 09290105.7
(22) Date de dépôt: 12.02.2009
(51) Int. Cl.: A61M 27/00, F16K 37/00, G01C 17/04

(54) **Dispositif de localisation mécanique et de lecture du réglage d'une valve ajustable**
Vorrichtung zu mechanischen Lokalisierung und zum Ablesen der Regulierung eines Justierventils
Device for mechanical localisation and reading the setting of an adjustable valve

(43) Date de publication de la demande: 18.08.2010
(73) Titulaire: Sophysa, 91893 Orsay Cedex (FR)
(72) Inventeur: Nègre, Philippe, 75116 Paris (FR); Moureaux, Christophe, 25000 Besancon (FR)
(74) Mandataire: Corret, Hélène

(56) Documents cités:
- EP-A- 0 723 783
- DE-A1- 10 235 353
- FR-A- 1 437 767
- FR-A- 2 858 239
- FR-A- 2 861 569
- US-A- 4 676 772

## Description

L'invention se rapporte à un dispositif de localisation et d'indication de réglage d'une valve ajustable.

Ce type de valve est utilisé dans le domaine médical comme, par exemple, dans le traitement de l'hydrocéphalie.

Cette maladie se caractérise par une hypersécrétion de liquide céphalo-rachidien (LCR), un défaut de résorption, ou une obstruction mécanique des voies de circulation, entraînant ainsi des troubles neurologiques et/ou moteurs chez le patient.

Ce type de valve est bien connu de l'Homme du Métier qui peut se référer notamment aux brevets EP 0 060 369 et EP 0 688 575 pour un exemple de réalisation d'une telle valve.

L'un des problèmes techniques que cette valve permet de résoudre est le réglage non invasif de la valve. En effet, s'il est nécessaire d'opérer le patient pour implanter un appareil de drainage comprenant la valve et des cathéters à l'endroit requis, il est important que des réglages puissent être réalisés après l'opération en réponse à une évolution positive ou négative de la maladie pour drainer plus ou moins de LCR hors des ventricules cérébraux ou de l'espace sous-arachnoïdien.

Pour résoudre ce problème, la valve est réglable grâce à la rotation d'un rotor muni d'éléments magnétiques. Avec un appareil de réglage également muni d'éléments magnétiques, il est possible d'entraîner le rotor de la valve en rotation, à travers la peau du patient, grâce au couplage magnétique des éléments magnétiques du rotor de la valve et de l'appareil de réglage.

Un premier système de l'État de la Technique comprend un instrument de localisation, un instrument d'indication de réglage et un instrument d'ajustement de réglage d'une valve implantée dans un patient. Ces instruments sont utilisés séquentiellement. L'instrument de localisation est placé au dessus de la valve après que celle-ci a été repérée par palpation de la peau du patient. L'instrument de localisation est alors placé sur la peau au dessus de la valve de telle sorte que celle-ci s'ajuste dans une lumière de l'instrument de localisation. Enfin, l'instrument d'indication de réglage de la valve est placé au dessus du centre de la valve ainsi repéré. Ce deuxième instrument comprend une aiguille aimantée pouvant pivoter selon un plan. Cette aiguille suit alors l'orientation des éléments magnétiques de la valve et indique alors le réglage. Le troisième instrument d'ajustement de réglage est placé à son tour sur la valve et manipulé de manière à autoriser un passage plus ou moins important du LCR.

Ce dispositif est peu pratique car il est possible de confondre la valve avec un réservoir sous-cutané ou avec une excroissance osseuse sans que l'instrument de localisation ne signale cette erreur, l'aiguille s'orientant selon le champ magnétique terrestre en l'absence d'autre champ magnétique. En outre, il est peu précis car la lumière de l'instrument de localisation présente nécessairement des dimensions suffisamment grandes pour ne pas tendre dangereusement la peau du patient et ris quer de la blesser. Ainsi, le centre de la valve n'est repéré qu'approximativement de sorte que des erreurs de lecture du réglage peuvent survenir. Enfin, le centre de la valve n'étant repéré que par insertion de la valve dans la lumière de l'instrument de localisation, il n'est pas possible de localiser puis de régler une valve inaccessible ou introduite plus profondément.

Pour remédier aux inconvénients des solutions précédentes, la Demanderesse a proposé, dans la Demande PCT/IB2007/003448, publiée sous le numéro WO 2009 034 410 A1 un dispositif de localisation mécanique et de lecture du réglage d'une valve magnétique ajustable, comprenant un compas magnétique et un sélecteur muni d'un repère d'alignement du compas par rapport à la direction de circulation du fluide à travers la valve.

Le compas comprend un plan de référence et un indicateur magnétique monté de manière à pivoter selon trois dimensions de l'espace sous l'effet du champ magnétique de la valve. Le réglage de la valve est représenté par l'angle formé, dans le plan de référence, entre l'indicateur magnétique et le repère d'alignement lorsque l'indicateur magnétique est positionné perpendiculairement au plan de référence, le compas étant alors centré sur le centre magnétique de la valve.

Ce dispositif permet ainsi, en une seule étape, une localisation précise du centre magnétique de la valve, déterminée par la position de l'indicateur magnétique perpendiculaire au plan de référence, et un repérage du réglage de la valve, déterminé par la position angulaire de l'indicateur magnétique par rapport à la direction indiquée par le repère d'alignement.

Le compas de ce dispositif est muni d'un aimant torique ou de deux aimants cubiques. L'agencement des pôles Nord et Sud n'est pas précisé dans cette Demande.

Cependant, il est précisé que ce dispositif peut être sensible au champ magnétique terrestre externe, différent de celui de la valve. En effet, la boussole s'aligne avec les aimants de la valve lorsqu'ils se trouvent en dessous car, à ce moment là, l'effet du champ magnétique des aimants est plus fort que celui du champ magnétique terrestre. Cependant, il y a une déviation non négligeable due à l'interaction du champ magnétique terrestre avec le champ magnétique des aimants.

Pour remédier à ce problème, il est proposé dans la Demande PCT/IB2007/003448 publiée sous le numéro WO 2009 034 410 A1, de munir le dispositif d'un blindage magnétique. Une telle solution a également été proposée dans le document FR 2 858 239. Ce blindage est un anneau circulaire réalisé dans un matériau magnétique entourant l'indicateur mobile du dispositif. Un dispositif de localisation d'objet implantés est connu du document EP O 723 783 A1.

Cependant, un tel blindage est complexe et coûteux. En outre, pour être le plus efficace possible, il faut entourer l'indicateur mobile avec une quantité importante de matériau magnétique, de manière à être le plus proche possible de cet indicateur. Or, ce faisant, on limite également la sensibilité de perception, par l'indicateur mobile, du champ magnétique émis par la valve.

Une autre solution pourrait consister à augmenter la puissance des aimants du compas et de la valve pour que le champ magnétique terrestre devienne négligeable.

Cependant, cette solution n'est pas envisageable car la valve équipée d'aimants trop puissants générerait des artéfacts inacceptables à l'IRM en distordant les images.

L'objectif de la présente invention vise à proposer un dispositif de localisation mécanique et de lecture du réglage d'une valve magnétique ajustable peu sensible au champ magnétique terrestre, de conception simple et économique, et permettant, en une seule étape, de localiser le centre magnétique de la valve et de déterminer le réglage de la valve.

A cette fin, l'invention a pour objet un dispositif de localisation mécanique et de lecture du réglage d'une valve magnétique ajustable pour le contrôle de la circulation d'un fluide dans une direction prédéterminée, comprenant un compas magnétique et un repère d'alignement du compas par rapport à la direction de circulation du fluide à travers la valve, dans lequel le compas magnétique comprend un plan de référence et un indicateur magnétique monté de manière à pivoter selon trois dimensions de l'espace sous l'effet du champ magnétique de la valve, le réglage de la valve étant représenté par l'angle formé, dans le plan de référence, entre l'indicateur magnétique et le repère d'alignement lorsque l'indicateur magnétique est positionné sensiblement perpendiculairement au plan de référence, le compas étant alors centré sur le centre magnétique de la valve, dans lequel l'indicateur magnétique est muni d'au moins un aimant apte à recevoir un champ magnétique et présentant une direction Nord-Sud, ledit au moins un aimant étant agencé de manière à ce que cette direction Nord-Sud soit sensiblement perpendiculaire au plan de référence lorsque l'indicateur magnétique est positionné sensiblement perpendiculairement au plan de référence.

Selon d'autres modes de réalisation :
- le dispositif peut comprendre deux aimants agencés en polarités inversées, l'un présentant une orientation Nord-Sud par rapport au plan de référence, et l'autre présentant une orientation Sud-Nord par rapport au plan de référence, lorsque le compas est centré sur le centre magnétique de la valve ;
- le compas peut présenter un moyen de détection agencé pour déterminer si un axe de l'indicateur magnétique est perpendiculaire au plan de référence ;
- le moyen de contrôle peut être une mire de centrage ;
- l'indicateur magnétique peut être agencé pour prendre appui sur un pivot permettant une rotation dans les trois dimensions de l'espace sous l'effet du champ magnétique de la valve.
- l'indicateur magnétique peut présenter un axe surmonté d'un élément d'indication de réglage s'étendant sensiblement perpendiculairement à l'axe, une partie d'appui sur le pivot et une partie de fixation de l'aimant ;
- la partie de fixation peut être agencée de manière opposée à l'élément d'indication de réglage par rapport à la partie d'appui ;
- l'indicateur magnétique peut comprendre un limiteur de pivotement ;
- le limiteur de pivotement peut présenter une forme en cage de rotule hémisphérique, hémi ovoïde ou tronconique ;
- un moyen de renfort peut être disposé dans la partie d'appui du pivot ;
- le moyen de renfort peut être en un matériau choisi parmi le rubis, le corindon et une céramique ;
- un anneau d'équilibrage peut être disposé autour de la partie de fixation de l'aimant de manière coaxiale à l'axe et opposée à l'élément d'indication de réglage par rapport à la partie d'appui sur le pivot ;
- le dispositif peut comprendre un sélecteur muni du repère d'alignement, le sélecteur et le compas étant séparables ; et/ou
- le dispositif peut comprendre une interface de lecture du réglage de la valve.

L'invention se rapporte également à un ensemble de localisation mécanique et de lecture du réglage d'une valve magnétique, comprenant un dispositif précédent ainsi qu'un programmateur apte à émettre un champ magnétique suffisant pour modifier le réglage de la valve.

La présente invention peut être utilisée dans toute application industrielle ou médicale dans laquelle une valve magnétique ajustable, destinée à contrôler la circulation d'un fluide, a été installée ou implantée avec une direction de circulation de fluide connue, mais de manière inaccessible directement, par exemple sous une plaque difficilement amovible, ou dans le corps d'un patient. Seule cette dernière application médicale de l'invention est décrite par la suite, sans constituer pour la Demanderesse un quelconque renoncement aux droits correspondant aux utilisations industrielles.

D'autres caractéristiques de l'invention seront énoncées dans la description détaillée ci-après faite en référence aux figures annexées qui représentent, respectivement :
- la figure 1, une vue schématique en perspective d'un dispositif selon l'invention de localisation et d'indication de réglage d'une valve magnétique à pression ajustable,
- la figure 2, une vue éclatée en perspective du compas magnétique du dispositif de la figure 1,
- les figures 3a et 3b, des vues éclatées en perspective de deux modes de réalisation d'un indicateur magnétique selon l'invention,
- les figures 4 et 5, des vues schématiques en coupe du compas de la figure 1 en utilisation,
- la figure 6, une vue éclatée en perspective d'un autre mode de réalisation d'un indicateur magnétique selon l'invention,
- la figure 7, une vue éclatée en perspective d'un compas magnétique incorporant l'indicateur magnétique de la figure 6,
- la figure 8, une vue schématique en coupe du compas de la figure 7,
- la figure 9, une vue éclatée en perspective d'un autre mode de réalisation d'un indicateur magnétique selon l'invention, et
- la figure 10, une vue schématique en coupe d'un compas muni de l'indicateur magnétique de la figure 9.

Dans l'ensemble du texte, le centre de la valve doit être compris comme le centre magnétique de la valve et non le centre géométrique.

On entend par « direction Nord-Sud » la direction de la droite D_{NS} passant par le pôle nord magnétique et par le pôle sud magnétique de l'aimant. Sur les dessins, le pôle Nord est hachuré et le pôle Sud est en blanc. Selon cette direction, l'aimant peut présenter soit une orientation Nord-Sud, soit une orientation Sud-Nord par rapport à un plan de référence.

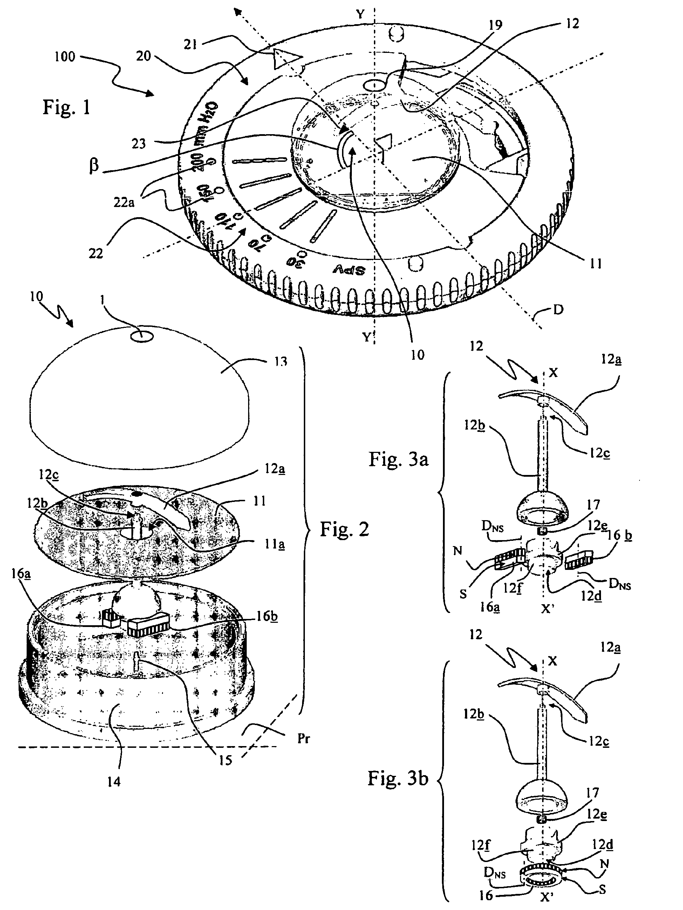

En se référant à la figure 1, un dispositif 100 de localisation et d'indication de réglage selon l'invention comprend un compas magnétique 10, et un sélecteur 20 muni d'une interface de lecture 22 et d'un repère d'alignement 21 du compas 10 par rapport à la direction D de circulation du fluide à travers la valve. Cette direction D est connue et prédéterminée lors de l'implantation de la valve (non représentée) chez un patient. Comme il sera précisé par le suite, il est préférable que le compas et le sélecteur puissent être séparables l'un de l'autre afin de pouvoir adapter un programmateur de valve sur le sélecteur à la place du compas, après avoir repéré le centre magnétique, et pouvoir ainsi régler la valve puisque le programmateur est localisé précisément au dessus du centre de la valve.

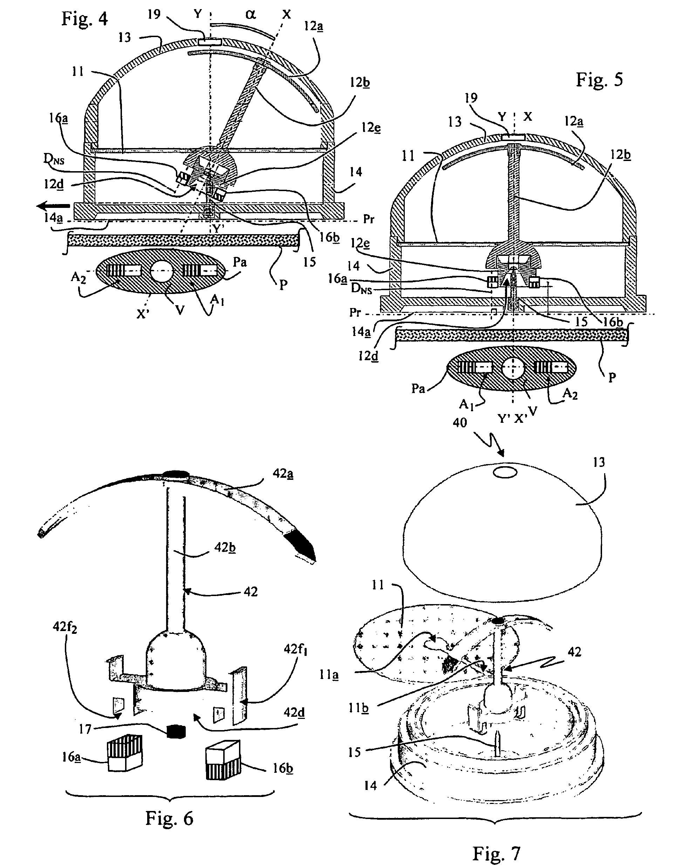

Plus particulièrement, le compas magnétique 10 comprend une plaque 11 disposée, de préférence, parallèlement à un plan de référence Pr (voir figure 4) et un indicateur magnétique 12 monté de manière à pivoter selon trois dimensions de l'espace sous l'effet d'un champ magnétique.

Dans un premier mode de réalisation d'un tel compas, tel qu'illustré par la vue éclatée de la figure 2, le compas comprend un dôme transparent 13 en forme de demi sphère et un socle 14 destinés à former ensemble un boîtier muni d'une base inférieure plane 14a. La plaque rapportée 11 est destinée à être fixée au socle 14 dans ou parallèlement au plan de référence Pr. L'indicateur magnétique 12 comprend un élément d'indication de réglage constitué par une aiguille 12a et un axe 12b destiné à être fixé perpendiculairement à l'aiguille 12a.

De plus, le socle 14 est muni d'un pivot 15 dont l'extrémité libre constitue un point d'appui pour l'axe 12b qui peut ainsi pivoter dans les trois dimensions de l'espace sous l'effet d'un champ magnétique.

La figure 3a illustre en détail la structure de l'indicateur magnétique 12. Dans ce mode de réalisation, l'indicateur magnétique 12 est démontable et composé de plusieurs pièces afin de pouvoir être inséré, lors du montage, dans l'ouverture 11 a de la plaque 11.

Ainsi, l'axe 12b comprend un moyen de fixation 12c à l'aiguille 12a de sorte que celle-ci surmonte l'axe 12b en s'étendant sensiblement perpendiculairement à lui.

L'indicateur magnétique 12 comprend également une partie d'appui 12d sur le pivot 15 située sur une pièce 12e destinée à être fixée sur l'axe 12b.

Cette partie d'appui 12d présente une forme en cage de rotule tronconique permettant à l'axe 12b de pivoter dans l'espace sans se désolidariser du pivot 15. Cette forme permet également de limiter la rotation tridimensionnelle de l'indicateur magnétique en fonction de son évasement.

Par ailleurs, l'indicateur magnétique 12 est muni d'une partie de fixation 12f pour deux aimants 16a et 16b, cette fixation étant également située sur la pièce 12e. La partie de fixation 12f est agencée de manière opposée à l'aiguille 12a par rapport à la partie d'appui 12d, c'est-à-dire qu'elle se situe à l'opposé de l'aiguille par rapport au point de pivotement.

Conformément à l'invention, les aimants 16a et 16b sont tels qu'ils présentent un pôle nord N et un pôle sud S agencés de telle sorte que la direction Nord-Sud, schématisée par les droites D_{NS} sur la figure 3a, soit parallèle à l'axe X-X' de l'indicateur magnétique. Autrement dit, lorsque le compas est centré sur le centre magnétique de la valve, l'axe X-X' de l'indicateur magnétique est sensiblement perpendiculaire au plan de référence Pr, et la direction Nord-Sud est également sensiblement perpendiculaire au plan de référence Pr.

Les deux aimants 16a et 16b sont préférentiellement agencés en polarités inversées : lorsque l'axe X-X' de l'indicateur magnétique est sensiblement perpendiculaire au plan de référence Pr, le pôle Nord de l'aimant 16a se trouve au dessus du pôle Sud par rapport au plan de référence Pr, et le pôle Sud de l'aimant 16b se trouve au dessus du pôle Nord par rapport au plan de référence Pr. Autrement dit, lorsque le compas est centré sur le centre magnétique de la valve, l'un des deux aimants (ici l'aimant 16a) présente une orientation Nord-Sud par rapport au plan de référence Pr, alors que l'autre aimant (ici l'aimant 16b) présente une orientation Sud-Nord par rapport au plan de référence Pr.

Dans la suite du texte, on appellera« disposition verticale » d'un aimant la disposition selon laquelle les pôles Nord et Sud sont superposés par rapport au plan de référence Pr lorsque l'axe X-X' de l'indicateur magnétique est sensiblement perpendiculaire au plan de référence Pr.

De manière surprenante, cette disposition verticale des aimants rend le dispositif de localisation mécanique et de lecture quasiment insensible au champ magnétique terrestre. Ainsi, l'indicateur magnétique 12 capte spécifiquement le champ magnétique des valves mais ne capte pas le champ magnétique terrestre. En outre, grâce à la disposition relative des deux aimants 16a et 16b, ils compensent l'effet d'un champ horizontal par leurs polarités inversées.

Au contraire, avec des aimants présentant une direction Nord-Sud horizontale, c'est-à-dire dont les pôles Nord et Sud sont disposés parallèlement au plan de référence du dispositif lorsque le compas est centré sur le centre magnétique de la valve, l'indicateur magnétique a une position instable et imprécise de plusieurs degrés, d'où la nécessité d'un blindage.

Un mode de réalisation alternatif de l'indicateur magnétique 12, illustré en figure 3b, prévoit un aimant torique 16 à la place des deux aimants 16a et 16b. Dans ce mode de réalisation, lorsque l'indicateur magnétique est sensiblement perpendiculaire au plan de référence Pr, le pôle Nord de l'aimant 16 se trouve au dessus du pôle Sud par rapport au plan de référence Pr. Il est évidemment possible d'envisager un montage dans lequel le pôle Sud se trouve au dessus du pôle Nord par rapport au plan de référence Pr.

Enfin, dans les deux modes de réalisation des figures 3a et 3b, un moyen de renfort 17 est préférentiellement disposé au fond de la partie d'appui 12d entre l'axe 12b et le pivot 15.

Ce moyen de renfort a pour fonction de renforcer le point de pivotement, et donc d'atteindre un pivotement de précision élevée. Il peut être constitué par une pièce en rubis, corindon, céramique, etc. placée au fond de la partie d'appui 12d.

Lors du montage, l'axe 12b est passé par l'ouverture 11a de la plaque 11 puis l'aiguille 12a est fixée à l'axe 12b par le moyen de fixation 12c. L'indicateur magnétique est alors posé sur le pivot 15 et la plaque 11 est positionnée sur le socle 14. Enfin le dôme transparent 13 est fixé au socle.

L'indicateur magnétique 12 est donc en appui sur le pivot 15, ce qui lui permet de pivoter dans les trois dimensions de l'espace sous l'effet d'un champ magnétique. Il convient donc de choisir une ouverture 11a suffisamment large pour permettre un pivotement angulaire maximum prédéterminé tout en étant plus étroite que la pièce 12d pour que la plaque 11 serve de garde fou pour empêcher l'indicateur magnétique de perdre son appui sur le pivot 15. Le fonctionnement de ce dispositif de localisation est illustré en figures 4 et 5.

Pour faciliter la détection du centre magnétique, le dôme du compas présente de préférence un moyen de détection agencé pour déterminer si l'axe X-X' de l'indicateur magnétique est perpendiculaire au plan de référence Pr. Ce moyen est, dans le mode de réalisation illustré, une mire de centrage 19 positionnée de telle sorte que l'axe X-X' de l'indicateur magnétique concorde avec l'axe Y-Y' du compas, perpendiculaire au plan de référence Pr, lorsque le compas est superposé à la valve et que l'indicateur magnétique se trouve précisément au dessus du centre de la valve.

L'utilisation du dispositif selon l'invention est décrite en référence aux figures 4 et 5, pour la localisation mécanique et la lecture du réglage d'une valve magnétique ajustable V. Le mode de réalisation illustré est celui de la figure 3a, c'est-à-dire que l'indicateur magnétique présente deux aimants 16a et 16 b agencés en polarités inversées.

L'utilisateur oriente le sélecteur 20 du dispositif 100 selon l'invention de sorte que le repère d'alignement 21 soit aligné avec la direction D de circulation du fluide à travers la valve. La direction D est déterminée lors de l'implantation de la valve et reste constante au cours de la vie du patient.

L'utilisateur maintient, en outre, le dispositif pour que le plan de référence Pr soit parallèle au plan Pa formé par des aimants de la valve. Le plan Pa est connu et prédéterminé lors de l'implantation de la valve qui est, généralement, implantée pour que le plan Pa soit sensiblement parallèle à la surface de la peau, avec un défaut d'assiette acceptable de quelques degrés qui peut être, par exemple de plus ou moins 5°. Il est donc souhaitable que le dispositif soit muni d'un moyen de positionnement par rapport au plan Pa. Par exemple, la face inférieure 14a du boîtier 14 peut être agencée parallèlement au plan Pr, de sorte qu'il suffit à l'utilisateur de placer le dispositif de manière à ce que la face inférieure 14a soit plaquée contre la peau du patient.

Puis l'utilisateur déplace l'ensemble compas/sélecteur au dessus de la valve en conservant, d'une part, l'alignement du dispositif par rapport à la direction (D) de circulation du fluide à travers la valve et, d'autre part, le parallélisme du plan de référence Pr par rapport au plan Pa.

L'utilisateur immobilise l'ensemble compas/sélecteur lorsque l'indicateur magnétique 12 est disposé sensiblement perpendiculairement par rapport au plan de référence Pr, c'est-à-dire lorsque l'utilisateur voit que l'axe de l'indicateur magnétique 12 se trouve dans la mire 19.

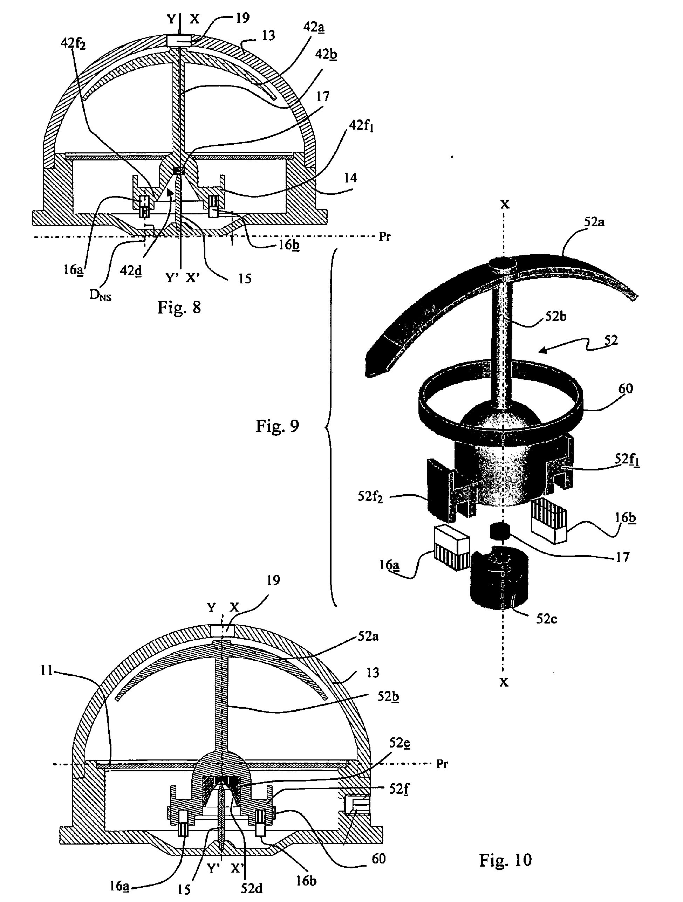

Lorsque l'indicateur magnétique 12 se positionne perpendiculairement au plan de référence Pr passant par la face inférieure 14a (figures 5 et 10), cela signifie qu'il se superpose à l'axe magnétique de la valve V, de sorte que la localisation du centre magnétique de la valve V est connue avec précision à travers la peau P.

Conformément à l'invention, les aimants 16a et 16b présentent un pôle nord N et un pôle sud S agencés de telle sorte que la direction Nord-Sud, schématisée par la droite D_{NS} sur les figures 4 et 5 soit sensiblement perpendiculaire au plan de référence Pr, lorsque le compas est centré sur le centre magnétique de la valvé (figure 5).

Ainsi, les aimants 16a et 16b de l'indicateur magnétique 12 s'alignent sur le champ magnétique généré par les aimants A₁-A₂ de la valve V, entraînant ainsi l'indicateur magnétique en rotation autour du point de pivot. Ainsi, tant que l'indicateur magnétique 12 ne se superpose pas précisément avec le centre magnétique de la valve V, il forme un angle α avec la perpendiculaire au plan de référence Pr.

Plus précisément, le pôle Sud de l'aimant 16a se place au dessus du pôle Nord de l'aimant A₁ de la valve V. De même, le pôle Nord de l'aimant 16b se place au dessus du pôle Sud de l'aimant A₂ de la valve V.

Les figures 4 et 5 sont schématiques et ne sont pas à l'échelle. En réalité, les aimants 16a et 16b sont agencés pour présenter un écartement semblable à l'écartement des aimants A₁-A₂ de la valve V pour optimiser leur interaction magnétique.

Grâce à cette disposition verticale des aimants, l'indicateur magnétique du dispositif selon l'invention capte spécifiquement le champ magnétique des aimants de la valve mais pas le champ magnétique terrestre. Quelle que soit l'orientation du champ magnétique terrestre, supposée localement horizontal, il n'a pas d'influence sur les aimants de l'indicateur magnétique. La localisation du centre magnétique de la valve V est donc connue avec précision à travers la peau P sans nécessité de blinder le dispositif.

Il n'est pas nécessaire d'avoir localisé la valve par palpation préalable pour pouvoir localiser son centre magnétique puisque le compas indique lui-même la direction à suivre. Par conséquent, le repérage de valves implantées profondément peut être effectué avec le dispositif selon l'invention. Cependant, lorsque le cas s'y prête, c'est-à-dire lorsque la valve est sous-cutanée, et pour gagner du temps, l'utilisateur peut effectuer une première localisation de la valve V par palpation dans la zone de localisation approximative de la valve V avant d'aligner le sélecteur avec la direction d'écoulement D.

L'utilisateur repère l'angle β formé, dans le plan de référence, entre l'indicateur magnétique 12 et la direction D indiquée par le repère d'alignement (figure 1).

Le réglage de la valve est alors représenté par cet angle β, c'est-à-dire par l'angle entre la projection orthogonale de l'aiguille 12a sur le plan de référence Pr et la direction D. Ainsi, comme illustré à la figure 1, il est possible de lire sur l'interface 22 que la valve est réglée pour laisser passer le fluide lorsque la pression est supérieure ou égale à 110 mm H₂O (soit environ 1078,73 Pa).

Le dispositif peut être compris dans un ensemble muni, en outre, d'un émetteur de champ magnétique, appelé programmateur, apte à émettre un champ magnétique suffisant pour modifier le réglage de la valve. Dans ce cas, après avoir localisé avec précision le centre magnétique de la valve et son réglage, l'utilisateur commande l'émission d'un champ magnétique suffisant pour modifier le réglage de la valve. Pour cela, dans le mode de réalisation préféré où le sélecteur et le compas sont séparables, l'utilisateur maintient le sélecteur 20 à l'endroit précis où le centre magnétique de la valve a été localisé, retire le compas 10 du sélecteur 20 et le remplace par un programmateur apte à s'ajuster dans le sélecteur 20. L'utilisateur peut alors effectuer un réglage approprié en fonction de la pression souhaitée de la valve.

Les figures 6 à 8 illustrent un autre mode de réalisation d'un compas magnétique 40 d'un dispositif de localisation et d'indication du réglage d'une valve magnétique selon l'invention.

Dans ce mode de réalisation, l'indicateur magnétique est constitué d'une seule pièce 42 (figure 6) présentant une partie 42a formant une aiguille d'indication de réglage, une partie 42b formant un axe, une partie d'appui 42d (figure 8) et une partie de fixation 42f₁-42f₂ de deux aimants 16a et 16b. Un rubis de renfort de pivotement 17 peut également être disposé à l'interface du pivot 15 et de la partie 42d.

Pour le montage de ce compas (figure 7), il convient de choisir une plaque 11 munie d'une encoche 11b permettant d'insérer l'axe 42b dans l'ouverture 11a.

Les autres étapes du montage sont identiques à celui du mode de réalisation de la figure 2.

Les figures 9 et 10 illustrent un autre mode de réalisation combinant les deux modes précédemment décrits, à savoir un indicateur magnétique en une pièce 52 comprenant une aiguille 52a, un axe 52b, une partie d'appui 52d localisée sur une pièce rapportée 52e, et une partie de fixation 52f₁-52f₂ pour des aimants 16a, 16b.

La pièce 52e a pour fonction de limiter l'angle de pivotement maximal de l'indicateur magnétique tout en limitant la quantité de rubis à utiliser uniquement au niveau du contact axe/pivot.

Par ailleurs, l'indicateur magnétique comprend un anneau d'équilibrage 60 permettant d'améliorer l'équilibre de l'indicateur magnétique 52 en contrebalançant le poids des parties de fixation 52f₁ et 52f₂ entre ces parties de fixation.

Cet anneau d'équilibrage 60 est disposé autour de la partie de fixation 52f₁-52f₂ de l'aimant, de manière coaxiale à l'axe 52b et opposée à l'élément d'indication de réglage 52a par rapport à la partie d'appui 52d sur le pivot 15.

De nombreuses variantes et alternatives peuvent être apportées sans pour cela sortir de l'invention et notamment :
- la boussole peut être utilisée pour déterminer le réglage de tout type de valve magnétique ajustable, tel que les valves à pression ajustable précédemment décrites ou les valves magnétiques à débit ajustable dans lesquelles la valve se ferme lorsqu'une quantité de fluide déterminée, calculée en fonction de la durée d'ouverture de la valve et du débit réglé, est passée à travers la valve,
- au lieu d'être monté en appui sur un pivot de rotation, l'indicateur magnétique peut être monté sur une rotule ou sur trois axes pivotants respectivement perpendiculaires, de manière à ce que l'indicateur puisse se positionner dans les trois dimensions de l'espace ;
- le plan de référence peut être constitué par la base du socle 14 et non par une plaque rapportée 11 ;
- le moyen de détection peut être un capteur émettant un signal sonore lorsque l'axe de l'indicateur magnétique est perpendiculaire au plan de référence ;
- le limiteur de pivotement peut présenter une forme en cage de rotule hémisphérique ou hémi ovoïde ;
- l'aimant peut être torique ou constitué de deux aimants ;
- la plaque peut comporter une graduation pour faciliter la lecture de réglage de la valve ;
- l'interface de lecture 22 comprend autant de repères 22a, d'indication de réglage que la va valve comprend de positions de réglage. Ainsi, dans la figure 1, l'interface de lecture est adaptée à une valve ayant jusqu'à cinq positions de réglage. Pour une valve ayant, par exemple, vingt-quatre positions de réglage, l'interface aura au moins vingt-quatre repères 22a ;
- l'indicateur magnétique peut comporter un moyen visuel 23 d'indication du repère 22a correspondant à la position de réglage de la valve. Ce repère peut être une simple marque telle qu'une flèche, un repère de couleur, ou une ligne, mais il également être matérialisé par une forme particulière de l'indicateur magnétique. Ce moyen visuel est particulièrement utile lorsque les repères 22a sont disposés de manière diamétralement opposée sur l'interface de lecture 22, car il permet de déterminer de manière univoque, entre deux repères 22a opposés, celui qui correspond à la position de réglage de la valve.

## Revendications

1. Dispositif de localisation mécanique et de lecture du réglage d'une valve (V) magnétique ajustable pour le contrôle de la circulation d'un fluide dans une direction prédéterminée (D), comprenant un compas magnétique (10) et un repère d'alignement (21) du compas (10) par rapport à la direction (D) de circulation du fluide à travers la valve (V), **caractérisé en ce que** le compas magnétique (10) comprend un plan de référence (Pr) et un indicateur magnétique (12, 42, 52) monté de manière à pivoter selon trois dimensions de l'espace sous l'effet du champ magnétique de la valve, le réglage de la valve étant représenté par l'angle (β) formé, dans le plan de référence, entre l'indicateur magnétique (12, 42, 52) et le repère d'alignement (21) lorsque l'indicateur magnétique (12, 42, 52) est positionné sensiblement perpendiculairement au plan de référence (Pr), le compas étant alors centré sur le centre magnétique de la valve, et **en ce que** l'indicateur magnétique (12, 42, 52) est muni d'au moins un aimant apte à recevoir un champ magnétique et présentant une direction Nord-Sud, ledit au moins un aimant étant agencé de manière à ce que cette direction Nord-Sud soit sensiblement perpendiculaire au plan de référence (Pr) lorsque l'indicateur magnétique (12) est positionné sensiblement perpendiculairement au plan de référence (Pr).

2. Dispositif selon la revendication 1, comprenant deux aimants (16a-16b) agencés en polarités inversées, l'un (16a) présentant une orientation Nord-Sud par rapport au plan de référence Pr, et l'autre (16b) présentant une orientation Sud-Nord par rapport au plan de référence Pr, lorsque le compas est centré sur le centre magnétique de la valve.

3. Dispositif selon l'une quelconque des revendications 1 ou 2, dans lequel le compas présente un moyen de détection agencé pour déterminer si un axe (X-X') de l'indicateur magnétique est perpendiculaire au plan de référence (Pr).

4. Dispositif selon la revendication 3, dans lequel le moyen de contrôle est une mire de centrage (19).

5. Dispositif selon l'une quelconque des revendications 1 à 4, dans lequel l'indicateur magnétique (12, 42, 52) est agencé pour prendre appui sur un pivot (15) permettant une rotation dans les trois dimensions de l'espace sous l'effet du champ magnétique de la valve.

6. Dispositif selon l'une quelconque des revendications 1 à 5, dans lequel l'indicateur magnétique (12, 42, 52) présente un axe (12b, 42b, 52b) surmonté d'un élément d'indication de réglage (12a, 42a, 52a) s'étendant sensiblement perpendiculairement à l'axe (12b, 42b, 52b), une partie d'appui (12d, 42d, 52d) sur le pivot et une partie de fixation (12f, 42f1-42f2, 52f) de l'aimant (16, 16a, 16b).

7. Dispositif selon la revendication 6, dans lequel la partie de fixation (12f, 42f1-42f2, 52f) est agencée de manière opposée à l'élément d'indication de réglage (12a, 42a, 52a) par rapport à la partie d'appui (12d, 42d, 52d).

8. Dispositif selon l'une quelconque des revendications 1 à 7, dans lequel l'indicateur magnétique (12, 42, 52) comprend un limiteur de pivotement (12e, 52e).

9. Dispositif selon la revendication 8, dans lequel le limiteur de pivotement présente une forme en cage de rotule hémisphérique, hémi ovoïde ou tronconique.

10. Dispositif selon l'une quelconque des revendications 1 à 9, dans lequel un moyen de renfort (17) est disposé dans la partie d'appui (12d, 42d, 52d) du pivot (15).

11. Dispositif selon la revendication 10, dans lequel le moyen de renfort (17) est en un matériau choisi parmi le rubis, le corindon et une céramique.

12. Dispositif selon l'une quelconque des revendications 1 à 11, dans lequel un anneau d'équilibrage (60) est disposé autour de la partie de fixation (12f, 42f1-42f2, 52f) de l'aimant (16, 16a, 16b) de manière coaxiale à l'axe (12b, 42b, 52b) et opposée à l'élément d'indication de réglage (12a, 42a, 52a) par rapport à la partie d'appui (12d, 42d, 52d) sur le pivot (15).

13. Dispositif selon l'une quelconque des revendications 1 à 12, comprenant un sélecteur (20) muni du repère d'alignement (21), le sélecteur (20) et le compas (10) étant séparables.

14. Dispositif selon l'une quelconque des revendications 1 à 13, comprenant une interface de lecture (22) du réglage de la valve (V).

15. Ensemble de localisation mécanique et de lecture du réglage d'une valve (V) magnétique, comprenant un dispositif selon l'une quelconque des revendications 1 à 14, ainsi qu'un programmateur apte à émettre un champ magnétique suffisant pour modifier le réglage de la valve.

## Claims

1. Device for mechanically locating and reading the setting of an adjustable-pressure magnetic valve (v) for controlling the flow of a fluid in a predetermined direction (D), comprising a magnetic compass (10) and an alignment mark (21) for aligning the magnetic compass (10) with respect to the direction (D) in which the fluid flows through the valve (V), **characterized in that** the magnetic compass (10) comprises a reference plane (Pr) and a magnetic indicator (12, 42, 52) which is mounted such that it can pivot in all three dimensions of space under the effect of the magnetic field of the valve, the valve setting being represented by the angle (ß) formed, in the reference plane, between the magnetic indicator (12, 42, 52) and the alignment mark (21) when the magnetic indicator (12, 42, 52) is positioned substantially perpendicular to the reference plane (Pr), the magnetic compass then being centered on the magnetic centre of the valve, and **in that** the magnetic indicator (12, 42, 52) is equipped with at least one magnet capable of receiving a magnetic field and having a north-south direction, the said at least one magnet being arranged in such a way that this north-south direction is substantially perpendicular to the reference plane (Pr) when the magnetic indicator (12) is positioned substantially perpendicular to the reference plane (Pr).

2. Device according to Claim 1, comprising two magnets (16a-16b) arranged with opposite polarities, one of them (16a) having a north-south orientation with respect to the reference plane (Pr) and the other (16b) having a south-north orientation with respect to the reference plane (Pr) when the compass is centered on the magnetic centre of the valve.

3. Device according to either one of Claims 1 and 2, in which the magnetic compass has a detection means designed to determine whether an axis (X-X') of the magnetic indicator is perpendicular to the reference plane (Pr).

4. Device according to Claim 3, in which the control means is a centring sight (19).

5. Device according to any one of Claims 1 to 4, in which the magnetic indicator (12, 42, 52) is designed to rest on a pivot (15) allowing it to rotate in all three dimensions of space under the effect of the magnetic field of the valve.

6. Device according to any one of Claims 1 to 5, in which the magnetic indicator (12, 42, 52) has a pin (12b, 42b, 52b) surmounted by a setting-indicating element (12a, 42a, 52a) running substantially perpendicular to the pin (12b, 42b, 52b), a part (12d, 42d, 52d) for resting on the pivot and a part (12f, 42f1-42f2, 52f) for attaching the magnet (16, 16a, 16b).

7. Device according to Claim 6, in which the attaching part (12f, 42f1-42f2, 52f) is arranged on the opposite side of the resting part (12d, 42d, 52d) to the setting-indicating element (12a, 42a, 52a).

8. Device according to any one of Claims I to 7, in which the magnetic indicator (12, 42, 52) comprises a pivot-angle restrictor (12e, 52e).

9. Device according to Claim 8, in which the pivot-angle restrictor is in the form of a hemispherical, hemiovoid or frustoconical swivel cage.

10. Device according to any one of Claims 1 to 9, in which a reinforcing means (17) is positioned in the resting part (12d, 42d, 52d) for the pivot (15).

11. Device according to Claim 10, in which the reinforcing means (17) is made of a material chosen from ruby, corundum and a ceramic.

12. Device according to any one of Claims 1 to 11, in which a balancing ring (60) is positioned around the part (12f, 42f1-42f2, 52f) for attaching the magnet (16, 16a, 16b) coaxial with the pin (12b, 42b, 52b) and on the opposite side of the part (12d, 42d, 52d) for resting on the pivot (15) to the setting-indicating element (12a, 42a, 52a).

13. Device according to any one of Claims 1 to 12, comprising a selector (20) equipped with the alignment mark (21), the selector (20) and the magnetic compass (10) being separable.

14. Device according to any one of Claims 1 to 13, comprising a reading interface (22) for reading the setting of the valve (V).

15. Assembly for mechanically locating and reading the setting of a magnetic valve (V), comprising a device according to any one of Claims 1 to 14 together with a programmer able to emit a magnetic field strong enough to alter the setting of the valve.

## Patentansprüche

1. Vorrichtung zur mechanischen Lokalisierung und zum Ablesen der Einstellung eines verstellbaren Magnetventils (V) zur Steuerung der Zirkulation eines Fluids in einer vorbestimmten Richtung (D), mit einem Magnetkompass (10) und einer Markierung (21) zur Ausrichtung des Kompass (10) in Bezug auf die Zirkulationsrichtung (D) des Fluids durch das Ventil (V), **dadurch gekennzeichnet, dass** der Magnetkompass (10) eine Bezugsebene (Pr) und eine magnetische Anzeige (12, 42, 52) aufweist, die so angebracht ist, dass sie unter der Wirkung des Magnetfelds des Ventils in drei Raumdimensionen schwenkt, wobei die Einstellung des Ventils durch den Winkel (β) dargestellt wird, der in der Bezugsebene zwischen der magnetischen Anzeige (12, 42, 52) und der Ausrichtmarkierung (21) gebildet ist, wenn die magnetische Anzeige (12, 42, 52) im Wesentlichen senkrecht zur Bezugsebene (Pr) angeordnet ist, wobei der Kompass dann auf die magnetische Mitte des Ventils zentriert ist, und die magnetische Anzeige (12, 42, 52) mit mindestens einem Magneten versehen ist, der ein Magnetfeld aufnehmen kann und eine Nord-Süd-Richtung aufweist, wobei der mindestens eine Magnet so angeordnet ist, dass diese Nord-Süd-Richtung im Wesentlichen senkrecht zur Bezugsebene (Pr) liegt, wenn die magnetische Anzeige (12) im Wesentlichen senkrecht zur Bezugsebene (Pr) angeordnet ist.

2. Vorrichtung nach Anspruch 1, mit zwei Magneten (16a-16b), die mit umgekehrten Polaritäten angeordnet sind, wobei der eine Magnet (16a) eine Nord-Süd-Ausrichtung in Bezug auf die Bezugsebene (Pr) und der andere Magnet (16b) eine Süd-Nord-Ausrichtung in Bezug auf die Bezugsebene (Pr) hat, wenn der Kompass auf die magnetischen Mitte des Ventils zentriert ist.

3. Vorrichtung nach einem der Ansprüche 1 oder 2, bei welcher der Kompass ein Erfassungsmittel aufweist, das so angeordnet ist, dass es bestimmt, ob eine Achse (X-X') der magnetischen Anzeige senkrecht zur Bezugsebene (Pr) liegt.

4. Vorrichtung nach Anspruch 3, bei der die Steuereinrichtung eine Zieleinrichtung (19) zum Zentrieren ist.

5. Vorrichtung nach einem der Ansprüche 1 bis 4, bei der die magnetische Anzeige (12, 42, 52) so angeordnet ist, dass sie an einem Drehzapfen (15) anliegt, der unter der Wirkung des Magnetfelds des Ventils eine Drehung in den drei Raumdimensionen ermöglicht.

6. Vorrichtung nach einem der Ansprüche 1 bis 5, bei der die magnetische Anzeige (12, 42, 52) eine Achse (12b, 42b, 52b), über der ein einstellungsanzeigendes Element (12a, 42a, 52a) angebracht ist, das sich im Wesentlichen senkrecht zur Achse (12b, 42b, 52b) erstreckt, einen Abschnitt (12d, 42d, 52d) zum Abstützen am Drehzapfen und einen Abschnitt (12f, 42f1-42f2, 52f) zur Befestigung des Magneten (16, 16a, 16b) aufweist.

7. Vorrichtung nach Anspruch 6, bei welcher der Befestigungsabschnitt (12f, 42f1-42f2, 52f) in Bezug auf den Abstützabschnitt (12d, 42d, 52d) entgegengesetzt zum einstellungsanzeigenden Element (12a, 42a, 52a) angeordnet ist.

8. Vorrichtung nach einem der Ansprüche 1 bis 7, bei der die magnetische Anzeige (12, 42, 52) einen Schwenkbewegungsbegrenzer (12e, 52e) umfasst.

9. Vorrichtung nach Anspruch 8, bei welcher der Schwenkbewegungsbegrenzer die Form eines halbkugelförmigen, halbovalen oder kugelstumpfförmigen Kugelgelenkkäfigs hat.

10. Vorrichtung nach einem der Ansprüche 1 bis 9, bei der ein Verstärkungsmittel (17) im Abstützabschnitt (12d, 42d, 52d) am Drehzapfen (15) angeordnet ist.

11. Vorrichtung nach Anspruch 10, bei der das Verstärkungsmittel (17) aus einem Material besteht, das aus Rubin, Korund und Keramik ausgewählt ist.

12. Vorrichtung nach einem der Ansprüche 1 bis 11, bei der um den Abschnitt (12f, 42f1-42f2, 52f) zur Befestigung des Magneten (16, 16a, 16b) ein Ausgleichsring (60) koaxial zur Achse (12b, 42b, 52b) und in Bezug auf den Abschnitt (12d, 42d, 52d) zur Abstützung am Drehzapfen (15) entgegensetzt zum einstellungsanzeigenden Element (12a, 42a, 52a) angeordnet ist.

13. Vorrichtung nach einem der Ansprüche 1 bis 12, mit einer Auswahleinrichtung (20), die mit der Ausrichtmarkierung (21) versehen ist, wobei die Auswahleinrichtung (20) und der Kompass (10) trennbar sind.

14. Vorrichtung nach einem der Ansprüche 1 bis 13, mit einer Schnittstelle (22) zum Ablesen der Einstellung des Ventils (V).

15. Baugruppe zur mechanischen Lokalisierung und zum Ablesen der Einstellung eines Magnetventils (V), mit einer Vorrichtung nach einem der Ansprüche 1 bis 14 und einer Programmiereinrichtung, die dazu geeignet ist, ein zur Änderung der Einstellung des Ventils ausreichendes Magnetfeld abzugeben.
